# EUROPEAN PATENT APPLICATION

(11) **EP 1 878 486 A1**
(43) Date of publication of application: **16.01.2008**
(21) Application number: 06117012.2
(22) Date of filing: 12.07.2006
(51) Int. Cl.: B01D 53/08, B01J 20/18, A61L 9/04, F24F 3/16

(54) **Air purification system for destruction of bacteria, virus and bacterial spores.**

(71) Applicant: Prozebo AB, 106 91 Stockholm (SE)
(72) Inventor: Andersson, Sten Eskil, 380 74 Löttorp (SE); Esmaeilzadeh, Saeid, 113 25 Stockholm (SE)
(74) Representative: Giavarini, Francesco

(57) **Abstract**

The invention concerns an air purification element and an air handling system comprising an air purification element, where the element comprising a zeolite or a mixture of zeolites, preferably hydrophobic, with micro-pores charged with an active element having affinity to the zeolite micro-pores and having antiseptic properties, thereby capable of killing bacteria and bacterial spores and inactivate virus, when the disinfectant is in direct contact with bacteria, bacterial spores or virus particles.

## Description

### TECHNICAL AREA

The invention is related to an air element and a system comprising an air purification element, where the air purification element comprises a disinfectant for the destruction of bacteria, virus and bacterial spores in air.

### BACKGROUND OF THE INVENTION

It is well-known to use various types of air filters for cleaning air from various contaminants like suspended particles, volatile organic compounds, bacteria, virus or bacterial spores.
W00212769 shows a filter system with several filter steps which can be connected to an air-conditioning system. One filter is a molecular sieve capable of attracting nitrogen and/or carbon dioxide and carbon monoxide containing molecules while allowing oxygen to pass. The molecular sieve may contain a hydrophilic zeolite of the general formula
M_{(x/n)} [(AlO₂)ₓ(SiO₂)_{y}] * zH₂O. The zeolite traps impurities and break bonds in compounds to release oxygen and retain carbon molecules. The filtered air may pass an additional fine filter for filtering out bacteria.
US 6190437 shows an air filter comprising several layers, whereof one filter is a disposable filter with a substrate of permeable nylon or the like impregnated with iodine resins in order to immobilize and attenuate airborne bacteria, fungi and viruses. Another filter comprises a zeolite impregnated with a medium for removing odour and unwanted gases from passing air.

Zeolite air handling systems for removing volatile organic compounds, VOC, are known. For example the Munters Zeol Rotor Concentrator. The rotor of the air handling system contains hydrophobic zeolites . Low concentration VOC laden air enters the rotor when an air stream pass trough a sector of the rotor. The VOC is adsorbed and clean air can be exhausted. The rotor is turning and in an isolated sector a small hot stream of clean air is drawn through the rotor desorbing the VOC from the zeolite and forming a concentrated VOC laden air stream. The regenerated zeolite is returned to the process as the rotor turns. The small VOC concentrated stream can be sent to an oxidizer or other apparatus for destroying the VOC.

There is generally a wide use of zeolites in various bed or filter configurations in such processes as purification of air from organic solvents including ethanol.

SE 505 274 can be mentioned as an example of a use of hydrophobic zeolites. It relates itself to the use of hydrophobic zeolites to eliminate preservatives, such as phenol or cresol, from medicine solution, for example insulin.

Several substances are known to have disinfection qualities, for example ethanol, iodine, phenol, cresol and hydrogen peroxide. Ethanol is widely used as a disinfectant because of its good effect with few or no side effects.

Air cleaning systems including filters for reducing the amount of bacteria in air are used in many types of air handling systems from air conditioning systems for temperature control to special systems used in hospitals or industry where the requirements for clean air or even sterile air are high. These filters are usually combined with other air cleaning filters for reducing the amount of contaminants in the air before the air stream passes the bacteria filter. The bacteria filter may be impregnated with a disinfectant like iodine or act as a micro sieve trapping the bacteria. A disadvantage with the known bacteria filters are that they get clogged and need to be exchanged at regular intervals, they are also expensive and the pressure drop over the bacteria filter is high. These filters are therefore unsuitable for use in many air handling systems.

There is a great demand for air purification systems which could also remove bacteria, virus and fungi from air flows. Such systems would find use in for example public buildings like hospitals, airports and railway stations or shops or offices. Also transport media like airplanes, trains, boats and busses as well as apartments and family houses can benefit from similar air filter or air handling systems.

### SUMMARY OF THE INVENTION

An object of the present invention is to provide an air purification element for destruction of bacteria, bacterial spores and virus from a stream of air.

Another object of the present invention is to provide an air purification element for destruction of bacteria, bacterial spores and virus from a stream of air, which is effective and does not cause a significant pressure drop in the system.

Another object of the invention is to form an air purification system comprising an active air purification element which can be used for effectively killing off bacteria, bacterial spores and inactivate virus from air flows, which is effective, have good air flow properties and allows for recovery of an active element used for killing off bacteria, bacterial spores and inactivate virus.

The objectives of the invention can be realised by having a purification element comprising a zeolite or a mixture of zeolites, preferably hydrophobic, with micro-pores charged with an active element having affinity to the zeolite micro-pores and having antiseptic properties, thereby capable of killing bacteria and bacterial spores and inactivate virus, when the disinfectant is in direct contact with bacteria, bacterial spores or virus particles.
The purification element can form a built-in part of or an add-on to an air handling system in order to add bacteria removal capabilities to a system or it can be an air purification element specifically used for removing bacteria etc from air in a confined space.

In an embodiment of the invention, an air handling system comprises a purification element comprising an active bed of zeolite pellets impregnated with a disinfectant like ethanol or iodine or a mixture thereof. The element has an opening for an incoming air flow, and an outlet leading to an absorption rotor. When air passes the purification element, bacteria will be effectively killed by the disinfectant vapours that are desorbed or released from the impregnated zeolite pellets. The air and vapour stream will be transported to an absorption rotor, which may also comprise zeolite.
The rotor will absorb all or nearly all solvents or iodine in the air. The rotor will also rotate and a warm air stream passing through a sector of the rotor can be used for regenerating the zeolite in the rotor.
The purification element can also form part of the rotor, as the rotor is built up by axial segments of zeolites and one or more segment is impregnated with one or more disinfectants.

The active part of the filter element can have various lengths dependent on time needed for securing the killing off of bacteria and bacterial spores or inactivation of viruses in a passing air stream.
The active part can also comprise sections with different types of disinfectant in order to more effectively kill off bacteria, bacterial spore or virus or to tailor make a purification element for killing off specific types of bacteria, bacterial spores or inactivation of specific viruses.

The use of zeolites in the purification element gives several advantages. One advantage is that the impregnation of the zeolites with disinfectant will lead to a slow release of the organic molecules making up the disinfectant. The zeolite can effectively be impregnated with a disinfectant like iodine, ethanol, peroxide or mixtures thereof, or with other disinfectants such as formaldehyde, chloroform, chlorine, chlorocarvacrol.
There is a large experience of using filters with zeolites for various air-cleaning purposes and these purification systems can be designed to have very good air flow properties.

Hydrophobic zeolites may be produced through the modification of synthetic hydrophilic zeolites, from which a large part of Al-ions is eliminated in order to make the zeolite become hydrophobic. This means that the chemical compositions of the hydrophobic zeolites in practice approach pure SiO₂. The hydrophobic zeolite silicalite is synthesized directly into the SiO₂ composition. The hydrophobic zeolite ZSM5 is very closely related in structure, composition and function to silicalite.

The hydrophobic zeolites which preferably can be used in the invention can be selected from a group containing silicalite or hydrophobic ZSM5, hydrophobic mordenite or hydrophobic zeolite Y.

The present invention will become apparent from the detailed description given below. Various changes and modifications within the spirit and scope of the invention will become apparent to those skilled in the art from this detailed description.

### BRIEF DESCRIPTIONS OF THE DRAWINGS.

Fig 1 shows an air handling system with an active air purification element and a rotor for regeneration of the active element.
Fig 2 shows a zeolite rotor containing active parts and regenerative parts.

### DETAILED DESCRIPTION OF THE INVENTION

With the definition of hydrophobic zeolites of the type (AlO₂)x(SiO₂)y as a negative network where x and y are integers, the ratio of y to x should exceed around 50 to make a hydrophobic zeolite.
The hydrophobic zeolites used for the active filter element are characterized by having a ratio of y to x of more 50 or more than 100 or 200 and preferably more than 900.

Pore sizes of the zeolites are typically smaller than 20 Å and the accessibility of the porous system is dimensionality dependent.
The hydrophobic varieties of zeolite Y (Toshoe Co., Japan) and mordenite can essentially be free from aluminium either through direct synthesis (silicalite) or by means of post synthetic manipulations (mordenite, zeolite Y). Zeolite Y and mordenite have pore sizes in the upper range, about 7.5 Å and 7.0 Å respectively while silicalite is a middle range zeolite with about 5.5 Å pore size. The porous systems in zeolite Y and silicalite are readily accessible due to the three dimensional arrangement of the channels; mordentite is somewhat less operative as the porous is one-dimensional.

High-silica zeolites have strong hydrophobic characteristics. They withstand high pressures and high temperatures without being altered.

The activation of the hydrophobic zeolites is done by adding up to 40 weight % of an active element.
In examples 1, 2 and 3 below, the efficiency of this method of destruction of bacteria and bacterial spores is demonstrated in experiments.

Hydrophobic zeolites are normally pre-treated before use at temperatures above 600 degrees centigrade.

### Example 1

A suspension of 25 ml, containing E. coli bacteria at 10⁶cells/ml, was prepared using 0.9% NaCl. The bacterial suspension was next pumped as an aerosol through a tube filled with hydrophobic zeolites, charged with 20 weight % iodine. All of the bacterial solution, meaning 2.5x10⁷ bacteria, had passed through the tube in about 30 minutes. To recognize surviving bacteria a filter (0.45 um pore size) was applied at the outlet. The filter was placed on a agar plate with Lauria Broth (LA plate) and the plate was incubated at 37°C over night. No bacterial colony could be detected on the filter placed on the LA plate. Since no viable bacteria could be demonstrated after passage through the column with iodine charged zeolite, this means that killing of the bacteria is total.

### Example 2

A suspension of 25 ml, containing E. coli bacteria at 10⁷ cells/ml, was prepared using 0.9% NaCl. The bacterial suspension was next pumped as an aerosol through a tube filled with hydrophobic zeolites, charged with 20 weight % ethanol. All of the bacterial solution, meaning 2.5x10⁸ bacteria, passed through the tube in either 1, 10, 20 or 30 minutes. To recognize surviving bacteria a filter (0.45 um pore size) was applied at the outlet. The filter was placed on a agar plate with Lauria Broth (LA plate) and the plate was incubated at 37°C over night. No bacterial colony could be detected on the filter placed on the LA plate. Since no viable bacteria could be demonstrated after passage through the column with ethanol charged zeolite, this means that killing of the bacteria is total.

### Example 3

A 10% suspension of Bacillus sp. spores (commerciably available from Gripen AB, Linköping, Sweden) was diluted 10⁻⁶ times in totally 25 ml of 0.9% NaCl solution. A sample of 0.1 ml from this suspension gives confluent bacterial growth if spread on an LA plate. All of the 25 ml volume of the spore suspension was passed as an aerosol through a column with hydrophobic zeolites charged with 20 weight % iodine. To recognize surviving bacteria a filter at the outlet was placed on a agar plate with Luria Broth (LA plate) and the plate was incubated at 37°C over night. No bacterial colony could be detected on the filter placed on the LA plate. Control experiments had shown that the bacterial spores are captured on the indicated filter and that the spores give rise to readily recognizable bacterial colonies if incubated at 37°C over night. Since no bacterial colonies could be demonstrated on the filter this means that spores and/or the bacteria that could have been hatched from them are totally killed as the result of passage through the column with iodine charged hydrophobic zeolites.

### Example 4

In order to demonstrate effective virus inactivation properties for the disinfectants suggested to be used in the active purification element, a stock of the E. coli bacteriovirus T4 with a titer of 10⁷ plaque forming units/ml (pfu) was incubated in iodine solution for 5 min. The plaque forming potential of the viral stock was completely eliminated, which supports efficient inactivation.

### Example 5

A stock of the E. coli bacteriovirus T4 was made by infecting a culture of growing E. coli with this phage. After the virus had lysed the bacterial culture the medium with its virus was treated with chloroform in order to kill any surviving bacteria. Control experiments confirmed that all bacteria were killed and the titer of the T4 virus was estimated to be 4.2x10¹⁰ plaque forming units/ml (pfu). The control experiments also showed that the viruses are small enough to pass through the used filters having a pore size of 0.45um or 0.2 um. However, an incubation of the virus (10⁷ pfu/ml) for 5 minutes in iodine solution completely eliminated the plaque forming potential of the virus stock. Therefore, iodine also inactivates virus in a very efficient manner.

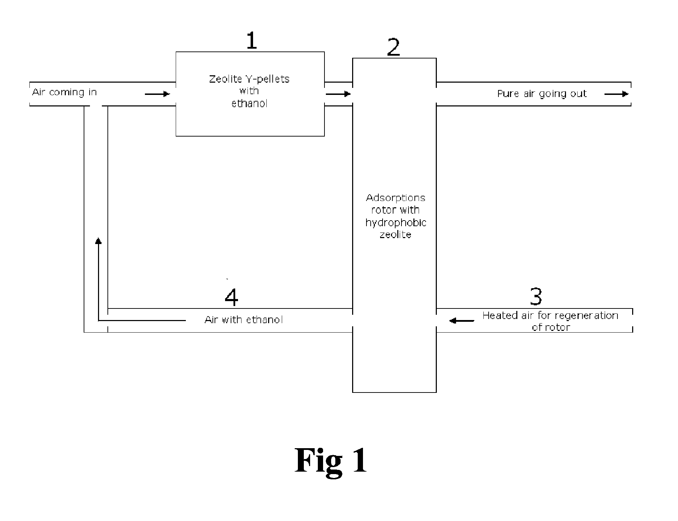

In the attached drawing (fig. 1) an air purification system is shown, comprising an active air purification element 1 comprising a bed of zeolits pellets charged with ethanol, so that the pellets are surrounded by deadly vapour of ethanol. A zeolite honeycomb absorption rotor 2 is placed downstream of the active air purification element 1 to absorb ethanol leaking from the active filter bed. Air from example a public building is let in at opening (Air coming in) and is led through the active air purification element 1 into the absorption rotor 2, where the air coming out from the active purification element is cleaned from ethanol and pure air pass out through an outlet (Pure air going out). Heated air is let in through an inlet 3 and is used for regenerating ethanol in conventional manner. The ethanol containing air is recirculated via a passage 4 and is mixed with the incoming air before passing through the active purification element to keep the vapour pressure of ethanol over the pellets.
The zeolite bed contains pellets loaded with ethanol, iodine or other substances efficient for destruction of bacteria, bacterial spores and virus. Microbes are destroyed by the vapour phase of ethanol from the mentioned zeolite pellets. The honeycomb absorption rotor 2 can contain zeolite of silicalite type.

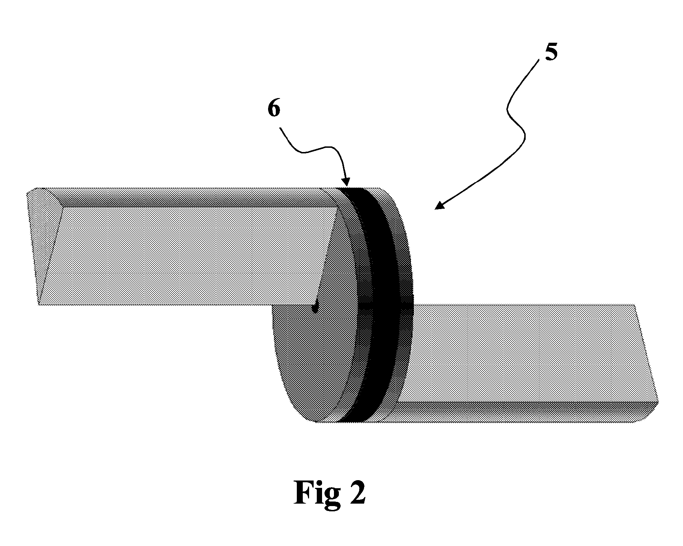

Fig 2 shows a hydrophobic zeolite honeycomb rotor 5 for destruction of bacteria and virus. in fig 1. Black region 6 marks content of absorbed ethanol. By having two inlets of air of opposite directions a more symmetrical distribution of ethanol in the black region is obtained. The efficiency of the destruction is augmented in this arrangement.

Embodiments of the present air purification system operating on full scale can be described in two steps, which are given in the figure descriptions above. Air to be cleaned from bacteria and virus is led into two constructions. The first construction is used to deliver active elements with desinfectionary quality, for example ethanol in form of vapour from a hydrophobic zeolite used for the destruction of bacteria and virus particles in the incoming air. Then a second construction downstream is needed to clean the air from the excess of ethanol released from the first construction. This is achieved by letting the air with the excess ethanol pass over another arrangement of a zeolite. The destruction of bacteria and virus particles occurs in an ordinary bed of zeolite pellets, preferably a hydrophobic zeolite of for example type Y. The ethanol is released with ordinary methods from the second construction and brought back into circulation by being absorbed in the bed of pellets.

## Claims

1. An air purification element for destruction of bacteria, bacterial spores and virus contained in air, **characterized by** that the purification element comprises zeolites with micro-pores charged with an active element having affinity to the zeolite micro-pores and having antiseptic properties and thereby being capable of killing bacteria and bacterial spores and inactivate virus.

2. An air purification element in accordance with claim 1 **characterized by** that the zeolite is an hydrophobic zeolite with a general structural formula (AlO₂)x(SiO₂)y as a negative network wherein a ratio y/x is at least 15, or more than 100, or more than 200 and preferably more than 900.

3. An air purification element in accordance with claim 1 or 2, **characterized by** that the zeolites contain up to 25 weight % of the active element.

4. An air purification element in accordance with claim 1 or 2, **characterized by** that the zeolites contain up to 40 weight % of the active element.

5. An air purification element in accordance with claim 1, 2, 3 or 4, **characterized by** that the micro pores of the zeolites have main cavities of uniform sizes smaller than 20 A.

6. An air purification element in accordance with any of the preceding claims, **characterized by** that the zeolites are selected from a group containing silicalite or hydrophobic ZSM5, hydrophobic mordenite or hydrophobic zeolite Y.

7. An air purification element in accordance with any of the preceding claims, **characterized by** that the active element is selected from a group of compounds including ethanol, iodine, hydrogen peroxide or mixtures thereof.

8. An air purification system, **characterized by** that the system comprises an air purification element in accordance with any of the preceding claims.

9. An air purification system in accordance with claim 8, **characterized by** that the system comprises a second purification element arrangement downstream of the first purification element, said second purification element, comprising hydrophobic zeolites for recovery of the active element.

10. An air purification system in accordance with claim 9, **characterized by** that the second air purification element arrangement is an absorption rotor.

11. An air purification system in accordance with claim 8, **characterized by** that the air purification element is a part of a rotor comprising hydrophobic zeolites.

12. An air purification system in accordance with claim 11, **characterized by** that the rotor is a honeycomb type of rotor having two inlets of air of opposite directions.

13. An air purification system in accordance with any of claims 8 - 12, **characterized by** that the active element is reused in the system.

14. An air purification system in accordance any of claims 8 -14, **characterized by** that all zeolites are pre-treated at temperatures above 600 degrees centigrade.
